(19) Europäisches Patentamt
European Patent Office
Office européen des brevets

(11) Publication number: **0 150 295**
**A2**

(12) # EUROPEAN PATENT APPLICATION

(21) Application number: **84113855.5**

(22) Date of filing: **16.11.84**

(51) Int. Cl.[4]: **C 07 D 223/10**, C 07 C 102/08

(30) Priority: **19.12.83 US 562675**

(43) Date of publication of application: **07.08.85**
**Bulletin 85/32**

(84) Designated Contracting States: **DE FR GB IT NL**

(71) Applicant: **ALLIED CORPORATION, Columbia Road and Park Avenue P.O. Box 2245R (Law Dept.), Morristown New Jersey 07960 (US)**

(72) Inventor: **Mares, Frank, c/o Allied Corporation P.O. Box 2245R, Morristown, N.J. 07960 (US)**
Inventor: **Tang, Reginald T. H., c/o Allied Corporation P.O. Box 2245R, Morristown, N.J. 07960 (US)**
Inventor: **Galle, James Edwin, c/o Allied Corporation P.O. Box 2245R, Morristown, N.J. 07960 (US)**
Inventor: **Federici, Rose Marie, c/o Allied Corporation P.O. Box 2245R, Morristown, N.J. 07960 (US)**

(74) Representative: **Baillie, Iain Cameron et al, c/o Langner Parry Isartorplatz 5, D-8000 München 2 (DE)**

(54) **Selective production of N-substituted amides by use of CU(O)/metallic oxides catalyst compositions.**

(57) This invention provides a process for the selective conversion of aliphatic and aromatic aminonitriles, such as epsilon-aminocapronitrile, or mixtures of a primary amine, $MNHR_2$, such as N-butylamine, and a nitrile, MCN, such as acetonitrile, into N-substituted amides, such as epsilon-caprolactam or N-butylacetamide, employing a Cu(O) catalyst in combination with oxides of a Group IVB, VB or VIB metal except chromium. The present invention, also provided a method of preparing a catalyst composition comprising Cu(O), substantially free of oxidized forms of Cu(O) and finely dispersed in oxides of a metal which is Ti, Zr, Hf, V, Nb, Ta, Mo or W, which comprises treating a substance comprising oxidized Cu and metal oxides with hydrogen at a pressure of at least about 100 kPa and at a temperature greater than about 250°C preferably about 350°–450°C for a time sufficient to produce a catalyst composition comprising Cu(O), substantially free of oxidized forms of Cu(O), finely dispersed in the metal oxides. The present invention also contemplates a catalyst composition comprising Cu(O), substantially free of oxides forms of Cu(O) and finely dispersed in oxides of Group IVB, VB or VIB metals except chromium.

EP 0 150 295 A2

# SELECTIVE PRODUCTION OF N-SUBSTITUTED AMIDES BY USE OF CU(O)/METALLIC OXIDES CATALYST COMPOSITIONS

## BACKGROUND OF THE INVENTION

This invention relates to the selective conversion of aliphatic and aromatic aminonitriles, such as epsilon-aminocapronitrile, or mixtures of a primary amine, $MNHR_2$, such as N-butylamine, and a nitrile, MCN, such as acetonitrile, into N-substituted amides, such as epsilon-caprolactam or N-butylacetamide, employing a Cu(O) catalyst in combination with oxides of a Group IVB, VB or VIB metal except chromium.

N-substituted amides, especially 5, 6 and 7 membered lactams, are important raw materials for nylon 4, 5 and 6.

U.S. Patent No. 2,357,484 (E.L. Martin) discloses a vapor phase process for preparing compounds containing the N-substituted amide group, for example, epsilon-caprolactam, by passing a vaporized mixture of water and an aliphatic amino-hydrogen containing aminonitrile, or a nitrile and primary or secondary amine over a dehydration catalyst such as activated alumina, silica gel or borophosphoric acid.

Japanese Patent Nos. 31542, 31543 and 31544 (I. Fujita et al., 1971) disclose methods for the preparation of epsilon-caprolactam by gas phase catalyzed reactions of a compound of the general formula $X(CH_2)_4COY$, wherein X is $CH_2OH$, CHO, CH(OR)(OR'), COOH, $COONH_4$, $CONH_2$ or COOR and wherein Y is OH, $ONH_4$, $NH_2$, $(C_1-C_4)$alkyl, cyclohexyl or OR, with ammonia and

hydrogen and in some cases steam, in the presence of a copper-metallic oxide catalyst which optionally may contain a third metal component to increase the catalytic activity. Japanese Patent No. 31542 (1971) discloses preparation and use of a copper-vanadium oxide catalyst; Japanese Patent No. 31543 (1971) discloses preparation and use of a copper-titanium oxide catalyst; and Japanese Patent No. 31544 (1971) discloses preparation and use of copper-molybdenum oxide catalyst. All the working examples of these three Japanese Patents disclose pretreating a catalyst bed of copper oxide-metal oxide mixture with a gaseous mixture of hydrogen and nitrogen, and teach taking caution not to let the catalyst bed temperature exceed 250°C.

The above-mentioned Japanese Patents disclose that a mixture of copper oxide-molybdenum oxide or other metal oxide be reduced by hydrogen at 150°-500°C before use in the preparation of epsilon caprolactam so as to form a catalyst that is believed to contain most of the copper in the metallic state. However, this isolated statement contradicts the teaching of the working examples of the above-mentioned Japanese Patents.

## SUMMARY OF THE INVENTION

In accordance with the broadest embodiment of the present invention, there is provided a process for selective preparation of N-substituted amides which comprises contacting, in the vapor phase, an aliphatic or aromatic aminonitrile having the formula $HR_1N\text{-}D\text{-}CN$ wherein D is a divalent organic moiety and, wherein $R_1$ is $(C_1\text{-}C_4)$alkyl or hydrogen, or mixtures of an amine having the formula $M\text{-}NR_2H$, wherein $R_2$ is $(C_1\text{-}C_4)$alkyl or hydrogen and a nitrile having the formula M-CN, wherein M is a monovalent organic moiety, with an effective amount of a Cu(O) catalyst, substantially free of oxidized forms of Cu, in combination with oxides of a metal which is Ti, Zr, Hf, V, Nb, Ta, Mo or W, at a temperature in the range of about 200° to about 400°C and at a hydrogen pressure in the range of about 0 to

about 500 kPa, in the presence of:

(a) ammonia in an amount equal to from 0 to about 50 mole percent of the molar amount of omega-aminonitrile or amine or nitrile present; and

(b) water in an amount from at least about 1.0 to about 50 times the molar amount of said aminonitrile or amine or nitrile present;

for a time sufficient to produce the corresponding N-substituted amide.

In accordance with another embodiment of the present invention, there is provided a process for the selective conversion of an aliphatic or aromatic aminonitrile into the corresponding lactam which comprises contacting, in the vapor phase, an aliphatic or aromatic aminonitrile having the formula $HR_1N$-D-CN wherein D is a divalent organic moiety, and wherein $R_1$ is $C_1$-$C_4$ alkyl or hydrogen, with an effective amount of a Cu(O) catalyst, substantially free of oxidized forms of Cu, in combination with oxides of a metal which is Ti, Zr, Hf, V, Nb, Ta, Mo or W, at a temperature in the range of about 200° to about 400°C and at a hydrogen pressure in the range of about 0 to about 500 kPa in the presence of:

(a) ammonia in an amount equal to from about 0 to about 50 mole percent of the aminonitrile present; and

(b) water in an amount from at least about 1.0 to about 50 times the molar amount of the aminonitrile present;

for a time sufficient to produce the corresponding lactam.

In accordance with still another embodiment of the present invention, there is provided a catalyst composition comprising Cu(O), substantially free of oxidized forms of Cu(O), finely dispersed in oxides of a metal which is Ti, Zr, Hf, V, Nb, Ta, Mo or W.

In accordance with still a further embodiment of the present invention, there is provided a method of preparing a catalyst composition comprising Cu(O), substantially free of oxidized forms of Cu(O) and finely dispersed in oxides of a metal which is Ti, Zr, Hf, V, Nb, Ta, Mo or W, which comprises treating a substance comprising oxidized Cu and metal oxides with hydrogen at a pressure of at least about 100 kPa and at a temperature greater than about 250°C for a time sufficient to produce a catalyst composition comprising Cu(O), substantially free of oxidized forms of Cu(O), finely dispersed in the metal oxides.

## DETAILED DESCRIPTION OF THE INVENTION OF THE PREFERRED EMBODIMENTS

The present invention is directed to the preparation of compounds containing N-substituted amides in high yield and high selectivity starting from aliphatic or aromatic aminonitriles or lower alkenyl or lower alkyl, primary or secondary organic amines admixed with lower alkenyl or lower alkyl nitriles. It is a special feature of the present invention that the high conversions of aminonitriles or admixtures of amines and nitriles may be achieved with high selectivity without the initial induction period of low selectivity experienced in the prior art by employing a copper catalyst substantially free of oxidized forms of copper (I) and copper (II). While the prior (Japanese References) art teaches pretreatment of the copper/metallic oxide catalysts, for example, copper/vanadium catalyst with hydrogen at a temperature not to exceed 250°C, we have surprisingly found that for pretreatment of the copper/vanadium oxide catalyst, a temperature in excess of 250°, preferably about 350° to 450°C is superior. By operating in accordance with the present invention, we have discovered that, at conversions greater than 90%, an aliphatic aminonitrile, such as epsilon-aminocapronitrile, is converted into the corresponding lactam with selectivities approaching 100% by use of an effective amount of a Cu(O) catalyst, substantially free of oxidized forms of copper, in combination with the oxides of a metal such as those of Group IVB, VB and VIB except

chromium at a temperature ranging from about 200° to 400°C in the presence of water vapor and optionally ammonia and/or hydrogen.

The catalyst compositions of the present invention comprising Cu(O), substantially free of oxidized forms of copper, and finely dispersed in the metal oxides are prepared by admixing a copper compound, such as inorganic copper nitrate, sulfate, carbonate, chloride, hydroxide or organic copper compounds, such as copper acetate or copper oxalate with a Group IVB metal compounds such as inorganic or organic compounds of titanium compounds, e.g., titanic acid, titanium or titanium oxalate, Group VB metal compounds, such as inorganic or organic vanadium, e.g, ammonium vanadate or vanadium acetate, or Group VIB inorganic or organic metal salts of molybdenum and tungsten but not chromium for time sufficient to form a solid which is pulverized dried and calcined in oxygen-containing gas such as air at elevated temperature. The pulverized oxidized mixture of metal oxides normally exists in the highest oxidation state of copper and metal oxides. It is a special feature of the present invention that the mixture of metal oxides is thereafter pretreated with hydrogen at a partial pressure of at least about 100 kPa and at a temperature greater than about 250°, preferably 350 to 450°C for a time sufficient to form a catalyst composition comprising Cu(O), substantially free of oxidized forms of copper (O) finely dispersed in the metal oxides. The preferred hydrogen pressure is in the range of about 100 to 2,000 kPa, preferably 100 to 2,000 kPa.

The reaction conditions in the pretreatment step such as reaction time and temperature are selected to produce a catalyst composition capable of high conversion, i.e., at least 90% of an aliphatic or aromatic aminonitrile or an admixture of amine and nitrile into the corresponding N-substituted amide at a selctivity (for amide) of at least 90%. For the copper/titanium

oxides catalyst, any temperature in the range of greater than about 250° to about 400°C and relatively long, e.g., about 57 hours reaction times produced a Copper/titanium oxides catalyst that exhibited more than 80% of selectivity to epsilon caprolactam at more than 90% conversion. For the copper/vanadium oxides catalyst, very long reaction times, e.g., 695 hours, and higher temperatures in the range of about 350°-400°C were required in the pretreatment step to produce a catalyst system exhibiting at least 90% selectivity at high conversion (about 98%). Compare Runs #1-3 of Tables Ia and Ib with Runs #9-14 of Tables IIa and IIb.

While the catalyst compositions of the present invention, in the form of finely dispersed copper in the metal oxides, may be prepared by the method described hereinabove, other methods such as treatment with strong reducing agents are also considered within the scope of the present invention.

Among the Group IVB compounds found useful in the process of preparing the catalysts composition of the present invention are inorganic and organic compounds of titanium, zirconium and hafnium, their 2,4-pentanedioates, carbonyls, $(C_1-C_{10})$alkoxides, (especially methoxides, ethoxides, propoxides and n-, sec- and tert-butoxides), halides (chlorides, bromides, fluorides and iodides), oxides, sulfates and mixed salts thereof, (especially oxide-halides, oxide-alkoxides, carbonylhalides, oxide-sulfates, and oxides-2,4-pentanedioates). Exemplary of the useful Group IVB compounds are the inorganic titanium materials, for example titanic acid, ammonium titanate, titanium nitrate, titanium halides such as titanium chloride, bromide or iodide, titanium sulfide, titanium sulfate, and organic titanium salts such as titanium $(C_1-C_{20})$alkanoate or alkanedioate, e.g., titanium acetate or titanium oxalate and titanium 2,4-pentanedioate.

Among the useful mixed salts are $Ti(CO)Cl_4$, $TiCl_3(OC_2H_5)$, $TiO[O_2C(CH_2)_3CO_2]$, $TiCl_2(OC_2H_5)_2$,

$TiCl(OC_2H_5)_3$, $TiOSO_4$, $TiOCl_2$, $ZrCl_3(O\text{-}i\text{-}C_3H_7)$, $ZrOCl_2$, $ZrCl_2(O\text{-}i\text{-}C_4H_9)_2$, $ZrOSO_4$, $Zr(CO)Cl_4$, $ZrO[O_2C(CH_2)_3CO_2]$, $HfCl(O\text{-}i\text{-}C_3H_7)_3$, $HfCl_2(O\text{-}t\text{-}C_4H_9)_2$, $HfCl_3(O\text{-}i\text{-}C_5H_{11})$, $HfOSO_4$, $Hf(CO)Cl_4$, $HfOCl_2$.

Among the Group VB compounds found useful in preparation in the catalysts composition of the present invention are inorganic and organic compounds of the vanadium, niobium and tantalum such as their nitrates, oxides, sulfates, halides (chlorides, bromides, iodides, or fluorides), carbonyls, 2,4-pentanedioates, $(C_1\text{-}C_{10})$-alkoxides, especially methoxides, ethoxides, propoxides and butoxides), $(C_1\text{-}C_{20})$alkanoates, and mixed salts such as oxide-halides, oxide-$(C_1\text{-}C_{10})$alkoxides, and carbonyl-halides, oxide-sulfates, and oxide-2,4-pentanedioates.

Among the Group VIB metal compounds of molybdenum and tungsten but not chromium found useful in the preparation of catalyst composition of the present invention are their nitrates, oxides, sulfates, halides (chlorides, bromides, iodides, or fluorides), carbonyls, 2,4-pentanedioates, $(C_1\text{-}C_{10})$alkoxides, (especially, methoxides, ethoxides, propoxides and n-, sec- and tert-butoxides), $(C_1\text{-}C_{20})$alkanoates, and mixed salts such as oxide-halides, oxide-$(C_1\text{-}C_{10})$alkoxides, oxide-sulfates, and carbonyl-halides, and oxide-2,4-pentanedioates.

Among the copper compounds found useful in the preparation of the catalyst composition of the present invention are inorganic copper compounds such as copper nitrate, copper sulfate, copper carbonate, copper chloride, copper hydroxide and organic copper compounds such as copper $(C_1\text{-}C_{20})$alkanoate, or alkanedioate, e.g., copper acetate, copper oxalate and other organic salts of copper.

The preferred catalyst composition of the present invention comprise Cu(O) substantially free of oxidized forms of copper Cu(O) finely dispersed in metal oxides or a metal oxide such as those of titanium, vanadium and molybdenum while a metal oxide such as $TiO_2$ or $V_2O_3$ or $MoO_3$ may be used, mixed oxides of titanium such as

$TiO/TiO_2/Ti_2O_3$ may conveniently be employed. The catalyst compositions of the present invention are selective for the formation of N-substituted amides and lactams only when they are subjected to pretreatment at a temperature of greater than about 250°, preferably in the range of about 350° to 450°C and at a hydrogen pressure of at least about 100 kPa for a time sufficient for the catalyst composition to lose the capability to catalyze hydrogenation required in the case of catalyst systems disclosed by the Japanese Patent Nos. 31542-44 (1971) to I. Fugita et al.

The process of the present invention for the highly selective production of acylic and cyclic N-substituted amides is conveniently operated by placing the catalyst compositions of the present invention in the form of pellets conveniently ground to a 20/30 mesh in a flow reactor. The activation or pretreatment of the catalyst compositions of the present invention by their reduction to copper Cu(O) in substantially free of oxidized forms of copper is conveniently effected in the flow reactor used for the preparation of N-substituted amides.

Temperatures in the range of about 200° to 400°C, preferably about 250° to about 350°C are adequate for the preparation of N-substituted amides. Contact times are not critical. Contact times of about 1 to about 5 seconds are preferred.

The reaction for the preparation of N-substituted amides may be conducted in the absence or in the presence of hydrogen. Hydrogen pressures in the range of about 0 to about 500 kPa and especially 50 to about 200 kPa are preferred. Ammonia may be present in an amount equal to about 0 to about 50 mol percent of the aminonitrile such as epsilon-aminocapronitrile or lower alkyl or alkenyl amines or lower alkenyl or lower alkyl nitriles. Water may be present in the reaction mixture in an amount from at least about 1.0 to about 50 times the molar amount of aminonitrile or amine or nitrile present.

Water in combination with primary or secondary aminonitrile or a mixture of a lower alkenyl or alkyl primary or secondary amine and a lower alkenyl or lower alkyl nitrile is introduced in the form of vapor into the reaction zone optionally together with gaseous ammonia and hydrogen. Preferably the process of the present invention is carried out at a hydrogen pressure in the range of about 50 to about 200 kPa, so as to maintain the copper catalysts in the copper zero state, substantially free of oxidized forms of copper thereby achieving high conversions and almost a 100% selectivity to the N-substituted amide for a long time.

The aliphatic or aromatic aminonitriles useful in the process of the present invention have the formula $HR_1N-D-CN$ wherein D is a divalent organic moiety having the formula:

$$[-(CHR')_m-X_p-(CHR'')_m-]$$

wherein X is O or NR' or ortho-phenylene, wherein R' and R" are independently hydrogen or $(C_1-C_8)$alkyl or $(C_1-C_8)$alkenyl or $(C_1-C_8)$alkyloxyalkyl, and wherein n + m = 3, 4, or 5 and wherein p is 0 or 1 and wherein the corresponding N-substituted amide is a lactam and wherein $R_1$ is $(C_1-C_4)$alkyl or hydrogen.

The orthophenylenes have the formula:

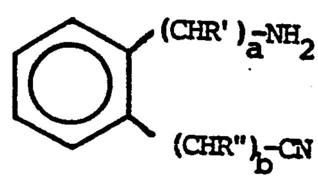

wherein R' and R" are hydrogen, $C_1-C_3$ alkyl and wherein a+b are selected so that a+b = 1-3.

Among the amino nitriles found especially useful in the process of the present invention are $H_2N-(CH_2)_3-CN$, $H_2N-(CH_2)_4-CN$, $H_2N-(CH_2)_5CN$, $H(CH_3)N-(CH_2)_3-CN$, $H(C_2H_5)N-(CH_2)_3-CN$, $H(n-C_3H_7)N-(CH_2)_3-CN$, $H(i-C_4H_9)N-(CH_2)_3-CN$, $H(CH_3)N-(CH_2)_4-CN$, $H(C_2H_5)N-(CH_2)_4-CN$, $H(i-C_3H_7)N-(CH_2)_4-CN$,

$H(n-C_4H_9)N-(CH_2)_4-CN$, $H(CH_3)N-(CH_2)_5-CN$, $H(C_2H_5)N-(CH_2)_5-CN$, $H(i-C_3H_7)N-(CH_2)_5-CN$ and $H(n-C_4H_7)N-(CH_2)_5-CN$, $H_2N-(CH_2)_2-O(CH_2)_6-CN$,

$$H_2N-(CH_2)_3-\overset{CH_3}{\overset{|}{N}}-(CH_2)_2-CN, \quad H_2HCH_2CH_2-\overset{CH_2CHCCH_3}{\overset{|}{CH}}-CN$$

and orthophenylenes such as $o-H_2N-(CH_2)_2-C_6H_4-CH_2-CN$, $o-H_2N-CH_2-C_6H_4-CN$ or $o-H_2N-CH_2-CH(CH_3)-C_6H_4-CH_2CN$ or amino alkyloxy alkylnitriles such as

$$H_2N-\underset{H}{\overset{CH_3}{C}}-CH_2O-\overset{CH_3}{C}HCH_2CN$$

Other aminonitriles obvious in view of this disclosure are considered within the scope of the present invention so long as the aliphatic or aromatic aminonitrile and corresponding lactam have vapor pressure sufficient to remain in the gaseous state under the conditions of the present invention. The preferred aminonitriles are $H_2N-(CH_2)_nCN$ wherein n is 3, 4 or 5.

The lower alkyl and lower alkenyl amines found useful in the present invention are primary and secondary amines having the formula $MNHR_2$ wherein M is a monovalent organic moiety independently selected from $(C_2-C_8)$alkenyl, $(C_1-C_8)$alkyl, or N, N di $(C_1-C_8$ alkyl) amino $(C_1-C_8)$alkyl or $(C_1-C_8)$alkyloxy $(C_1-C_8)$alkyl and wherein $R_2$ is $(C_1-C_4)$alkyl, such as methyl, ethyl, n- and i-propyl and n-, sec- and tert-butyl, or hydrogen.

Exemplary lower alkyl and lower alkenyl amines found particularly useful in the process of the present invention are methyl, ethyl, propyl, butyl, pentyl, hexyl, heptyl, octyl, and isomers thereof; ethenyl, propenyl, butenyl, pentenyl, hexenyl, heptenyl, octenylamines and isomers thereof; N,N(di-lower alkyl)amine such as N-N-dimethylaminomethylamine; and lower alkyl oxyalkylamines such as 3-(ethyloxy)propyl amine, 2-(isobutyloxy)ethylamine.

The lower alkyl and lower alkenyl nitriles found useful in the present invention have the formula MCN wherein M is a monovalent organic moiety as defined

hereinabove. Exemplary nitriles found useful in the present invention include: $CH_3CN$, $CH_3CH_2CN$, $(CH_3)_2CHCN$, $CH_3(CH_2)_2CN$ $CH_3(CH_2)_3CN$, $CH_3CH_2C(CH_3)HCH_2CN$, $CH_3(CH_2)_5CN$, $CH_3(CH_2)_6CN$, $CH_3(CH_2)_7CN$ and $CH_3(CH_2)_3$-$C(CH_2CH_3)HCN$, $CH_3CH=CHCH_2CN$, $CH_2=CHCH_2CN$, $CH_3CH_2CH=CHCN$, $CH_2=CHCH_2CH_2CN$, $CH_3CH_2CH=CH(CH_2)_3CN$, $CH_3(CH_2)_2O(CH_2)_3CN$ and $CH_3OCH_2CH_2CN$.

## Experimental

### Example 1

### Cu/Ti Catalyst Preparation

A catalyst composed of copper and titanium was prepared by modifying a procedure in Japanese Patent No. 31543 (1971).

$Ti_2(C_2O_4)_3$ (50 g) was dissolved into 92 mL of $H_2O$. To this was added the solution prepared by dissolving $Cu(NO_3)_2 \cdot 3H_2O$ (48 g) into 92 mL of $H_2O$. The mixture was stirred at room temperature for about 30 minutes. The solid was then filtered. The resultant cake was dried at 90°C, and 40 g of a light blue solid was obtained. It was pulverized and sieved through an 80 mesh sieve and then calcined in a stream of air at the following time-temperature program:

| | |
|---|---|
| 30°C→70°C | 15 minutes |
| 70°C→350°C | 40 hours |
| 350°C | 8 hours |

Elemental Analysis: Cu - 27%, Ti - 8%

X-ray Analysis (ESCA): $Cu^{+2}$; $Ti^{+4}$

### Example 2

### Conversion of ε-Aminocapronitrile to ε-Caprolactam

This Example illustrates the procedure for the ε-aminocapronitrile conversion to ε-caprolactam using the Cu-Ti catalyst prepared as described in Example 1. The catalyst after the calcination step was pelletized (4000 psig) and ground to 20/30 mesh. The resulting catalyst (6 mL, 5.52 g) was placed in a glass flow reactor on top of a glass fritted disc. Quartz chips (2 g), used for vaporization and dispersion of the reactants during the run, were placed on top of the catalyst. The catalyst

was reduced using a gas at low $H_2/N_2$ ratio which was raised gradually to 100% $H_2$ while the catalyst temperature was also gradually raised from room temperature to 250°C. The final conditions were maintained for a short duration (usually 1 hour), at which point the temperature was increased to the reaction temperature of 300°C and the appropriate gas and reactant liquid feed were started.

The gas flow and feed conditions were:

$H_2$ - 15 gas mL/min (52.3%)
$NH_3$ - 4 gas mL/min (14.0%)
6-aminocapronitrile - 5.9 x $10^{-3}$ liquid mL/min (3.8%)
$H_2O$ - 6.9 x $10^{-3}$ liquid mL/min (30%)
Contact time - 6.6 sec.

The gas flows were regulated by appropriate Tylan flow controllers. The liquid reactants were delivered by Sage syringe pumps. The products were collected in round-bottom flasks (kept at Dry Ice temperatures) with gas release side-arms. Product samples were diluted with methanol, and triglyme was used as gc standard for quantitative analysis. The results are summarized in Tables Ia and Ib.

The catalyst was removed from the reactor after the run and was found to be Cu(O) by X-ray diffraction; no Cu(I) or Cu(II) was found.

Tables Ia and b

Conversion of ε-aminocapronitrile into ε-caprolactam in the Presence of Cu*/Ti Catalyst of Example 2 at 290°C

Table Ia

Reaction Conditions

| Run # | Time (hr.) | $H_2$ (%) | $N_2$ (%) | $NH_3$ (%) | $H_2O$ (%) | Substrate (%) |
|---|---|---|---|---|---|---|
| 1[a] | 6.1 | 76.7 | 0 | 6.8 | 14.6 | 1.8 |
| 2[b] | 13.8 | 76.7 | 0 | 6.8 | 14.6 | 1.8 |
| 3 | 36.8 | 76.7 | 0 | 6.8 | 14.6 | 1.8 |
| 4 | 59.7 | 52.3 | 0 | 14.0 | 30.0 | 3.8 |
| 5 | 82.6 | 52.3 | 0 | 14.0 | 30.0 | 3.8 |
| 6 | 103.0 | 0 | 52.3 | 14.0 | 30.0 | 3.8 |
| 7 | 126.0 | 0 | 52.3 | 14.0 | 30.0 | 3.8 |
| 8 | 129.0 | 52.3 | 0 | 14.0 | 30.0 | 3.8 |

* Cu(O) after Reaction (X-ray Diffraction Analysis)

a. Reaction Temperature was 300°C

b. Reaction Temperature was 280°C

c. ε-aminocapronitrile

After each run, the organic substrate feed valve was closed and the catalyst was treated in same apparatus for 6-10 hours under conditions of previous run. Then, the valve for organic substrate feed was opened and the new run was started. Thus after Run #5, the organic substrate feed was stopped and the catalyst was treated at 290°C for 10 hours with $H_2$ and $NH_3$ and $H_2O$. Between Runs #6 and 7, $N_2$ and $NH_3$ and $H_2O$, but not $H_2$ or ε-aminocapronitrile were fed to the reactor. Thus, the sum of the Run times reflects the total contact time of catalyst with the organic substrate.

Table 1b

Products of Reaction

| Run # | Conversion (%) | Material Balance (%) | Lactam[d] (%)S | Imine[e] (%)S | Cpone[f] (%)S | Unknown |
|---|---|---|---|---|---|---|
| 1 | 100.0 | 56.8 | 38.5 | 0 | 5.4 | 6.0 |
| 2 | 98.9 | 81.7 | 74.6 | 0 | 1.7 | 2.0 |
| 3 | 96.0 | 96.4 | 82.3 | 0 | 4.9 | 0 |
| 4 | 95.7 | 118.6 | 102.0 | 0 | 4.0 | 9.8 |
| 5 | 95.4 | 98.0 | 86.0 | 0 | 1.6 | 10.8 |
| 6 | 93.6 | 100.0 | 91.1 | 0 | 1.1 | 2.9 |
| 7 | 90.0 | 100.0 | 98.0 | 0.8 | 0 | 2.2 |
| 8 | 90.7 | 105.0 | 102.0 | 0 | 0.9 | 2.9 |

d ε-caprolactam

e hexamethyleneimine

f cyclopentanone

## Example 3

### Cu/V Catalyst Preparation

A catalyst composed of copper and vanadium was prepared by modifying a procedure in Japanese Patent No. 31542-1971.

$Cu(NO_3)_2 \cdot 3H_2O$ (130 g) was dissolved into 250 mL of $H_2O$. To this was added the solution prepared by dissolving $NH_4VO_3$ (70 g) into $H_2O$ (at 80°C) containing $NH_4OH$ (15 mL). The solid thus obtained was then filtered. The residue was dried at 110°C for about 10 hours, pulverized and calcined in a stream of air at the following time-temperature program:

| | | | |
|---|---|---|---|
| 30°C→120°C | 15 minutes | 220°C→320°C | 30 minutes |
| 120°C | 1 hour | 320°C | 1 hour |
| 120°C→220°C | 30 minutes | 320°C→335°C | 10 minutes |
| 220°C | 1 hour | 335°C | 3.5 hours |

Elemental Analysis: Cu - 28%, V - 28%

X-ray Analysis (ESCA): $Cu^{+2}$; $V^{+5}$.

## Example 4

The procedure and apparatus of Example 2 were employed to convert ε-aminocapronitrile into ε-caprolactam in the presence of the Cu/V catalyst prepared as described in Example 3. The results are summarized in

Tables IIa and IIb.

Tables IIa and b

Conversion of ε-aminocapronitrile into ε-caprolactam in the Presence of Cu*/V Catalysts of Example 3 at 290°C

Table IIa

Reaction Conditions

| Run # | Time (Hr) | $H_2$ (%) | $N_2$ (%) | $NH_3$ (%) | $H_2O$ (%) | Substrate[a] (%) |
|---|---|---|---|---|---|---|
| 9 | 25.5 | 54.7 | 0.0 | 14.6 | 27.2 | 3.5 |
| 10 | 50.5 | " | " | " | " | " |
| 11 | 76.5 | " | " | " | " | " |
| 12 | 132.5 | " | " | " | " | " |
| 13 | 185.0 | " | " | " | " | " |
| 14 | 230.5 | " | " | " | " | " |
| 15 | 257.0 | " | " | " | " | " |

* Cu(O) after reaction by X-ray diffraction

a. ε-aminocapronitrile

Table IIb

Products of Reaction

| Run # | Conversion (%) | Material Balance (%) | Lactam[b] %S | Imine[c] %S | Cpone[d] %S | Unknowns |
|---|---|---|---|---|---|---|
| 9 | 97.8 | 79.4 | 29.2 | 2.5 | 24.9 | 22.3 |
| 10 | 99.5 | 94.7 | 51.6 | 2.0 | 19.1 | 21.3 |
| 11 | 99.1 | 92.8 | 56.1 | 1.7 | 16.2 | 18.2 |
| 12 | 99.5 | 93.7 | 57.3 | 1.4 | 15.9 | 18.3 |
| 13 | 99.4 | 88.5 | 67.2 | 1.4 | 8.2 | 11.4 |
| 14 | 99.3 | 108.0 | 87.7 | 6.1 | 5.0 | 9.3 |
| 15 | 98.3 | 107.0 | 90.6 | 0.6 | 3.3 | 12.7 |

b. ε-caprolactam

c. hexamethyleneimine

d. cyclopentanone

Examples 5 and 6

These Examples (runs #16, 17) illustrate reduction of the copper in the Cu/V catalyst of Example 3 to Cu(O) and use of same for conversion of ε-aminocapronitrile into ε-caprolactam at high temperature. After the catalyst and quartz chips were placed in the reactor of

Example 4 as before, the catalyst pre-treatment was initiated using a gas at low $H_2/N_2$ ratio which was raised gradually to 100% $H_2$ while the catalyst temperature was also gradually raised from room temperature to 400°C. These conditions (400°C and 100% $H_2$) were maintained for a period of over two days. The reactor temperature was then adjusted to 290°C for reaction. This high temperature catalyst pre-treatment rendered the catalyst highly selective to caprolactam formation immediately. Copper, at the zero valent oxidation state, was determined for this activated catalyst at the end of the reaction by X-ray diffraction. The results are summarized in Tables IIIa and b.

Tables IIIa and b

Conversion of ε-aminocapronitrile into ε-caprolactam in the presence of prereduced Cu(O)/V catalyst of Example 5 at 290°C

Table IIIa

Reaction Conditions

| Run # | Time (Hr) | $H_2$ (%) | $N_2$ (%) | $NH_3$ (%) | $H_2O$ (%) | Substrate[a] (%) |
|---|---|---|---|---|---|---|
| 16 | 20 | 54.7 | 0 | 14.6 | 27.2 | 3.5 |
| 17 | 41 | " | " | " | " | " |

a. ε-aminocapronitrile

Table IIIb

Reaction Products

| Run # | Conversion (%) | Material Balance (%) | Lactam[b] (%S | Imine[c] %S | Cpone[d] %S | Unknowns |
|---|---|---|---|---|---|---|
| 16 | 92 | 111 | 110 | 0.35 | 0.5 | 0 |
| 17 | 82.2 | 95.4 | 93.8 | .2 | 0 | 0.4 |

b. ε-caprolactam

c. hexamethyleneimine

d. cyclopentanone

These Examples demonstrate that a higher pretreatment temperature is needed for Cu/V system to produce a catalyst that is highly selective for formation of lactam. In Example 4, the Cu/V catalyst only achieved 90% selectivity to lactam after 695 hours (cumulative reaction time of runs 9-14). It is believed that once the catalyst is taken to the selective state (as shown by % selectivity and run times in Run #14 & 15 and 16 and 17), the catalyst will maintain that state provided at least some hydrogen is present in the reaction zone.

Example 7

Cu/Mo Catalyst Preparation

A catalyst composed of copper and molybdenum was prepared by modifying the procedures found in Japanese Patent Nos. 31544 (1971) and 4794 (1973).

$(NH_4)_6Mo_7O_{24} \cdot 4H_2O$ (213 g) was dissolved into $H_2O$ (50°C) containing conc. $NH_4OH$ (250 mL) and diluted to 900 mL. The solution was heated at 65°C for about 10 minutes.

To this was added the solution prepared by dissolving $Cu(NO_3)_2 \cdot 3H_2O$ (263 g) into $H_2O$ (80°C) and diluted to 900 mL. The resulting mixture was allowed to stand for 20 minutes. The solid was filtered and washed twice with $H_2O$ (50 mL). It was placed in an oven at 110°C for 8 hours, pulverized and calcined in a stream of air at the following time-temperature program:

| | |
|---|---|
| 30°→100°C | 30 minutes |
| 100°C→120°C | 10 minutes |
| 120°C | 1 hour |
| 120°C→320°C | 60 minutes |
| 320°C | 1 hour |
| 320°C→335°C | 10 minutes |
| 335°C | 3.5 hours |

Elemental Analysis: Cu - 19.7%, Mo - 51%

X-ray Analysis (ESCA): $Cu^{+2}$; $Mo^{+6}$

Example 8

The procedure and apparatus of Example 2 were employed to convert ε-aminocapronitrile into ε-caprolactam in the presence of the Cu/Mo catalyst prepared as described in Example 7. The results are summarized in Tables IVa and b.

Tables IVa and b

Conversion of ε-aminocapronitrile into ε-caprolactam in the presence of Cu*/Mo catalysts of Example 7 at 290°C

Table IVa

Reaction Conditions

| Run # | Time (hr.) | $H_2$ (%) | $N_2$ (%) | $NH_3$ (%) | $H_2O$ (%) | Substrate[a] (%) |
|---|---|---|---|---|---|---|
| 18 | 24.5 | 51.1 | 0 | 13.6 | 32.5 | 2.8 |
| 19 | 46.0 | " | " | " | " | " |
| 20 | 92.0 | " | " | " | " | " |
| 21 | 150.0 | " | " | " | " | " |

Table IVb

Reaction Products

| Run # | Conversion (%) | Material Balance (%) | Lactam[b] %S | Imine[c] %S | Cpone[d] %S | Unknowns |
|---|---|---|---|---|---|---|
| 18 | 62.1 | 82.4 | 56.2 | 1.5 | 4.1 | 7.1 |
| 19 | 36.3 | 97.1 | 74.4 | 0.7 | 6.2 | 6.8 |
| 20 | 25.4 | 108.0 | 108.0 | 1.2 | 7.4 | 11.0 |
| 21 | 26.5 | 105.0 | 100.0 | 2.2 | 7.0 | 5.6 |

*Cu(O) after reaction by X-ray diffraction

a. ε-aminocapronitrile

b. ε-caprolactam

c. hexamethyleneimine

d. cyclopentanone

EXAMPLES 9-18

In the following Examples the catalyst and process of Examples 5 and 6 are repeated in the same apparatus excepting that approximately equimolar mixtures of amine, $MNHR_2$, and nitrile, MCN is used as indicated in the following table.

| Example | $MNHR_2$ | MCN |
|---|---|---|
| 9 | $CH_3NH_2$ | $CH_3CH{=}CHCH_2CN$ |
| 10 | $C_2H_5NH_2$ | $CH_3(CH_2)_7CN$ |
| 11 | $(C_2H_5)_2NH$ | $CH_3(CH_2)_6CN$ |
| 12 | $n\text{-}C_3H_7N(CH_3)H$ | $CH_3(CH_2)O(CH_2)_3CN$ |
| 13 | $CH_2{=}CHCH_2NH_2$ | $CH_3O(CH_2)_2CN$ |
| 14 | $n\text{-}C_5H_{11}NH_2$ | $(CH_3)_2CHCN$ |
| 15 | $i\text{-}C_6H_{13}NH_2$ | $CH_2{=}CH(CH_2)_2CN$ |
| 16 | 3-pentenyl$NH_2$ | $CH_3(CH_2)_5CN$ |
| 17 | $n\text{-}C_8H_{17}NH_2$ | $CH_3CN$ |
| 18 | $C_2H_5O(CH_2)_3NH_2$ | $C_2H_5CN$ |

Similar results to those reported in Examples 5-8 are expected.

It will be apparent that various modifications are possible within the spirit of the invention and therefore it is understood that the invention shall not be limited by the above Examples but only insofar as it becomes necessary by the limitations recited in the appended claims taken in light of the prior art.

0150295

We Claim:

1. A process for selective preparation of N-substituted amides which comprises contacting, in the vapor phase, an aliphatic or aromatic aminonitrile having the formula $HR_1N\text{-}D\text{-}CN$ wherein D is a divalent organic moiety and, wherein $R_1$ is $(C_1\text{-}C_4)$alkyl or hydrogen, or mixtures of an amine having the formula $M\text{-}NR_2H$, wherein $R_2$ is $(C_1\text{-}C_4)$alkyl or hydrogen, and a nitrile having the formula M-CN wherein M is a monovalent organic moiety, with an effective amount of a Cu(O) catalyst, substantially free of oxidized forms of Cu, in combination with oxides of a metal which is Ti, Zr, Hf, V, Nb, Ta, Mo or W, at a temperature in the range of about 200° to about 400°C and at a hydrogen pressure in the range of about 0 to about 500 kPa, in the presence of:

(a) ammonia in an amount equal to from 0 to about 50 mole percent of the molar amount of said aminonitrile or amine or nitrile present; and

(b) water in an amount from at least about 1.0 to about 50 times the molar amount of said ammonitrile or amine or nitrile present for a time sufficient to produce the corresponding N-substituted amide.

2. The process of claim 1 wherein the hydrogen pressure is maintained at a value sufficient to maintain the Cu(O) catalyst substantially free of Cu(I) and/or Cu(II).

3. The process of claim 2 wherein the hydrogen pressure is maintained at a value in the range of about 50 to about 200 kPa.

4. The process of claim 1 wherein the metal oxides are oxides of titanium.

5. The process of claim 1 wherein said Cu(O) catalyst in combination with said metal oxides is pretreated with hydrogen at a temperature greater than 250°C.

6. The process of claim 1 wherein the divalent organic moiety D has the formula

$$[\text{-}(CHR')_n\text{-}X_p\text{-}(CHR'')_m\text{-}]$$

wherein X is O or NR' or ortho-phenylene,

wherein R' and R" are independently hydrogen or $(C_1-C_8)$alkyl or $(C_1-C_8)$alkenyl or $(C_1-C_8)$alkyloxyalkyl, and

wherein n + m = 3, 4, or 5 and wherein p is 0 or 1 and wherein the corresponding N-substituted amide is a lactam; and wherein the monovalent organic moiety M is independently $(C_1-C_8)$alkyl, or $(C_2-C_8)$alkenyl, or N, N[di$(C_1-C_8)$alkyl]amino $(C_1-C_8)$alkyl or $(C_1-C_8)$alkyloxy$(C_1-C_8)$alkyl.

7. A process for the selective conversion of aliphatic and aromatic aminonitriles into the corresponding lactams which comprises contacting, in the vapor phase, an aliphatic or aromatic aminonitrile having the formula $HR_1N-D-CN$ wherein D is a divalent organic moiety and, wherein $R_1$ is $(C_1-C_4)$alkyl or hydrogen, with an effective amount of a Cu(O) catalyst, substantially free of oxidized forms of Cu, in combination with oxides of a metal which is Ti, Zr, Hf, V, Nb, Ta, Mo or W, at a temperature in the range of about 200° to about 400°C and at a hydrogen pressure in the range of about 0 to about 500 kPa in the presence of:

(a) ammonia in an amount equal to about 0 to about 50 mole percent of the aminonitrile present; and

(b) water in an amount from at least about 1.0 to about 50 times the molar amount of the aminonitrile present

for a time sufficient to produce the corresponding lactam.

8. The process of claim 7 wherein the aminonitrile is epsilon-aminocapronitrile.

9. A method of preparing a catalyst composition comprising Cu(O), substantially free of oxidized forms of Cu(O) and finely dispersed in oxides of a metal which is Ti, Zr, Hf, V, Nb, Ta, Mo or W, which comprises treating a substance comprising oxidized Cu and metal oxide with hydrogen at a pressure of at least about 100 kPa and at a temperature greater than about 250°C for a time sufficient to produce a catalyst composition

comprising Cu(O), substantially free of oxidized forms of Cu(O), finely dispersed in the metal oxide.